# EUROPÄISCHE PATENTSCHRIFT

(11) **EP 1 579 854 B1**
(45) Veröffentlichungstag und Bekanntmachung des Hinweises auf die Patenterteilung: **03.08.2011**
(21) Anmeldenummer: 05004962.6
(22) Anmeldetag: 07.03.2005
(51) Int. Cl.: A61K 9/70, A61K 47/34

(54) **Dermales oder transdermales therapeutisches System mit Matrixbestandteil aus nachwachsendem Rohstoff**
Dermal or transdermal therapeutic system comprising a matrix with a renewable raw material
Système thérapeutique dermal ou transdermal comprénant une matrice constituée par un matériau recroissant

(30) Priorität: 10.03.2004 DE 102004011686
(43) Veröffentlichungstag der Anmeldung: 28.09.2005
(73) Patentinhaber: Acino AG, 83714 Miesbach (DE)
(72) Erfinder: Salman, Nouha, Dr., 80799 München (DE); Fischbacher, Eva Maria, 83075 Bad Feilnbach (DE)
(74) Vertreter: Forstmeyer, Dietmar

(56) Entgegenhaltungen:
- WO-A1-95/30411
- WO-A2-98/22097
- US-A- 4 668 232
- US-A1- 2003 152 611
- LISKA ROBERT: JOURNAL OF POLYMER SCIENCE. PART A. POLYMER CHEMISTRY, 40(10), 1504-1518, 19 REFS. ISSN: 0887-624X CODEN: JPLCAT, 2002, XP009051658
- SINTOV A. ET AL: "Enzymatic cleavage of disaccharide side groups in insoluble synthetic polymers: a new method for specific delivery of drugs to the colon" BIOMATERIALS, , 14(7), 483-490, 21 REFS. ISSN: 0142-9612, 1993, XP002338744
- DEPPE O ET AL: "Surface functionalization of styrene/butyl acrylate latex with a water-soluble monosaccharide monomer. Synthesis and morphology of the polymer particles" POLYMERS FOR ADVANCED TECHNOLOGIES, (JUN 2003) VOL. 14, NO. 6, PP. 409-421. ISSN: 1042-7147. PB - JOHN WILEY & SONS LTD, THE ATRIUM, SOUTHERN GATE, CHICHESTER PO19 8SQ, W SUSSEX, ENGLAND., 2003, XP001177215

## Beschreibung

Aufgabe der Erfindung ist es, ein dermales oder transdermales therapeutisches System mit einer Polymermatrix vorzusehen, zu deren Herstellung nicht ausschließlich von erschöpfbaren Rohstoffquellen ausgegangen wird.

Gemäß der Erfindung wird die ihr zugrundeliegende Aufgabe durch ein dermales oder transdermales therapeutisches System mit einer Polymermatrix gelöst, bei der das Polymere wiederkehrende Einheiten mit einem Bestandteil aus einem nachwachsenden Rohstoff aufweist bzw. unter Verwendung von Monomeren mit einem Bestandteil (Monomerbestandteil) aus einem nachwachsenden Rohstoff aufgebaut ist.

Liska Robert, Journal Of Polymer Science. Part A. Polymer Chemistry, 40(10), 1504-1518, 19, 2002 offenbart kopolymerisierbare Methakrylderivate von Deoxyglucitol

Sintov A. et al: Biomaterials, , 14(7), 483-490, 21 beschreibt Kopolymere von Glukopyranosyl -1-methakrylamido-1-deoxy-glucitol mit Methakrylsäure, sowie deren Verwendung zur gezielten Freigabe von Wirkstoffen im Dickdarm.

Deppe O et al: Polymers For Advanced Technologies, 14 (6), 409-421, 2003 offenbart MAG-Kopolymere mit Methyl-und Ethylakrylat zur Herstellung von Latexmatrizen.

WO 98/22097 A offenbart Mischungen aus polaren Polymeren, dessen Monomere Alginate, Dextran, Zellulose, Mannitol oder Xylitol sein können, und Polyacrylate zur Herstellung von z.B, transdermale Verabreichungsformen. Jedoch werden keine zuckeralkoholderivatisierte Polyacrylate eingesetzt.

Eine Ausführungsform der Erfindung betrifft ein dermales oder transdermales therapeutisches System mit einem Monomerbestandteil aus einem Zuckeralkohol oder einem Zuckeralkoholderivat nach Anspruch 1 als nachwachsendem Rohstoff.

Bei dem erfindungsgemäßen dermalen oder transdermalen therapeutischen System bildet
- der Zuckeralkohol als 1-Desoxy-1-amino-Zuckeralkohol oder
- das Zuckeralkoholderivat als 1-Desoxy-1-amino-Zuckeralkoholderivat
die Ester- oder Amidogruppen einer polymerisierten Acrylsäure.

Ferner betrifft eine Ausführungsform der Erfindung ein dermales oder transdermales therapeutisches System mit Methacrylsäure als Acrylsäure.

Bei dem erfindungsgemäßen dermalen oder transdermalen therapeutischen System kann es sich bei dem Zuckeralkoholderivat um ein Disaccharid handeln.

Ferner betrifft eine Ausführungsform der Erfindung ein dermales oder transdermales therapeutisches System mit Lactitol als Disaccharid.

Bei dem erfindungsgemäßem dermalen oder transdermalen therapeutischen System können die Hydroxygruppen des Zuckeralkohols oder des Zuckeralkoholderivats, soweit sie nicht mit der polymerisierten Acrylsäure verknüpft sind, mit Schutzgruppen versehen sein.

Ferner können bei dem erfindungsgemäßen dermalen oder transdermalen therapeutischen System die Schutzgruppen durch Acylgruppen gebildet werden, insbesondere durch Acetylgruppen.

Ferner kann bei dem erfindungsgemäßen dermalen oder transdermalen therapeutischen System die Acrylsäure mit einem oder mit mehreren Comonomeren polymerisiert sein.

Ferner betrifft eine Ausführungsform der Erfindung ein dermales oder transdermales therapeutisches System mit einem Acrylat als Comonomerem.

Ferner betrifft eine Ausführungsform der Erfindung ein dermales oder transdermales therapeutisches System mit Ethylacrylat, Butylacrylat, Hexylacrylat oder 2-Ethylhexylacrylat als Comonomerem.

Ferner betrifft eine Ausführungsform der Erfindung ein dermales oder transdermales therapeutisches System mit einer Matrix aus
Poly-[(1-desoxy-1-methacrylamido-glucitol)-co-(ethylacrylat)],
Poly-[(1-desoxy-1-methacrylamido-glucitol)-co-(butylacrylat)],
Poly-[(1-desoxy-1-methacrylamido-glucitol)-co-(hexylacrylat)],
Poly-[(1-desoxy-1-methacrylamido-glucitol)-co-(2-ethylhexylacrylat)],
Poly-[(penta-O-acetato-1-desoxy-1-methacrylamido-glucitol)-co-(ethylylacrylat)],
Poly-[(penta-O-acetato-1-desoxy-1-methacrylamido-glucitol)-co-(butylacrylat)],
Poly-[(penta-O-acetato-1-desoxy-1-methacrylamido-glucitol)-co-(hexylacrylat)],
Poly-[(penta-O-acetato-1-desoxy-1-methacrylamido-glucitol)-co-(2-ethylhexylacrylat)].
Poly-[(1-desoxy-1-methacrylamido-lactitol)-co-(ethylacrylat)],
Poly-[(1-desoxy-1-methacrylamido-lactitol)-co-(butylacrylat)],
Poly-[(1-desoxy-1-methacrylamido-lactitol)-co-(hexylacrylat)],
Poly-[(1-desoxy-1-methacrylamido-lactitol)-co-(2-ethylhexylacrylat)],
Poly-[(penta-O-acetato-1-desoxy-1-methacrylamido-lactitol)-co-(ethylylacrylat)],
Poly-[(penta-O-acetato-1-desoxy-1-methacrylamido-lactitol)-co-(butylacrylat)],
Poly-[(penta-O-acetato-1-desoxy-1-methacrylamido-lactitol)-co-(hexylacrylat)] und/oder
Poly-[(penta-O-acetato-1-desoxy-1-methacrylamido-lactitol)-co-(2-ethylhexylacrylat)].

Bei dem erfindungsgemäßen dermalen oder transdermalen therapeutischen System kann die Matrix zusätzlich einen oder mehrere Weichmacher umfassen.

Ferner kann das erfindungsgemäße dermale oder transdermale therapeutische System einen oder mehrere Weichmacher aus der durch Triethylenglykol (TEG), Triethylcitrat (TEC), Propylenglycol (PG), Glycerol (G), Triacetin (TriAc), Bis-(2-ethylhexyl)-adipat (EtHex), Diisobutyladipat (i-But) und Isopropylmyristat (IPM)
gebildeten Gruppe umfassen.

Ferner kann das erfindungsgemäße dermale oder transdermale therapeutische System neben der Matrix eine Abdeckfolie und gegebenenfalls eine Abziehfolie umfassen.

Ferner können bei dem erfindungsgemäßen dermalen oder transdermalen therapeutischen System die Abdeckfolie und/oder die Abziehfolie Folien auf Polyesterbasis sein.

Ferner können bei dem erfindungsgemäßen dermalen oder transdermalen therapeutischen System die Abdeckfolie und/oder die Abziehfolie Folien auf Basis von Polyethylentherephthalat sein.

Ferner kann bei dem erfindungsgemäßen dermalen oder transdermalen therapeutischen System die Matrix einen oder mehrere Wirkstoffe umfassen.

So kann bei dem erfindungsgemäßen dermalen oder transdermalen therapeutischen System die Matrix Ibuprofen als Wirkstoff umfassen.

Schließlich kann bei dem erfindungsgemäßen dermalen oder transdermalen therapeutischen System die Matrix neben den fakultativen Weichmachern einen oder mehrere übliche Hilfsstoffe umfassen.

### Beispiel 1

Es wurde eine Lösung von 1,0 g Poly-[(1-desoxy-1-methacrylamido-glucitol)-co-(butylacrylat)] in 1,5 g Ethanol mit einem Zusatz von 20 % Foral und 43 % Propylenglycol (PG) unter Erwärmen auf 80 °C gelöst. Foral war in 0,50 g Ethylacetat vorgelöst worden. Sofort danach wurde ein 75 µm dicker Polyesterfilm mit einseitiger Silikonisierung (Loparex FL 2000) beschichtet, wonach im Trockenschrank bei 50 °C 5 min lang getrocknet wurde. Die Kleberschicht wurde mit einem 15 µm dicken Polyethylenterephthalat-Film (Mitsubishi Polyester Film GmbH) kaschiert. Die Klebkraft war sehr gut.

### Beispiel 2

Es wurde eine Lösung von 1,0 g Poly-[(1-desoxy-1-methacrylamido-glucitol)-co-(butylacrylat)] in 1,5 g Ethanol mit einem Zusatz von 30 % Foral und 43 % Propylenglycol unter Erwärmen auf 80 °C gelöst. Foral war in 0,50 g Ethylacetat vorgelöst worden. Sofort danach wurde ein 75 µm dicker Polyesterfilm mit einseitiger Silikonisierung (Loparex FL 2000) beschichtet, wonach im Trockenschrank bei 50 °C 5 min lang getrocknet wurde. Die Kleberschicht wurde mit einem 15 µm dicken Polyethylenterephthalat-film (Hostaphan; Mitsubishi Polyester Film GmbH) kaschiert. Die Klebkraft war sehr gut.

### Beispiel 3

Es wurde eine Lösung von 1,0 g Poly-(1-desoxy-1-methacrylamido-lactitol-co-butylacrylat) in 2,0 g Ethanol mit einem Zusatz von 20 % Foral und 30 % Propylenglycol unter Erwärmen auf 80 °C gelöst. Foral war in 0,50 g Ethylacetat vorgelöst worden. Sofort danach wurde ein 75 µm dicker Polyesterfilm mit einseitiger Silikonisierung (Loparex FL 2000) beschichtet, wonach im Trockenschrank bei 50 °C 5 min lang getrocknet wurde. Die Kleberschicht wurde mit einem 15 µm dicken Polyethylenterephthalat-film (Hostaphan; Mitsubishi Polyester Film GmbH) kaschiert. Die Klebkraft war ausgezeichnet.

### Beispiel 4

Es wurde eine Lösung von 1,0 g Poly-(1-desoxy-1-methacrylamido-glucitol-co-butylacrylat) in 2,0 g Ethanol mit einem Zusatz von 20 % Foral und 30 % Glycerol unter Erwärmen auf 80 °C hergestellt. Foral war in 0,50 g Ethylacetat vorgelöst worden. Sofort danach wurde ein 75 µm dicker Polyesterfilm mit einseitiger Silikonisierung (Loparex FL 2000) beschichtet, wonach im Trockenschrank bei 50 °C 5 min lang getrocknet wurde. Die Kleberschicht wurde mit einem 15 µm dicken Polyethylenterephthalat-film (Hostaphan; Mitsubishi Polyester Film GmbH) kaschiert. Die Klebkraft war befriedigend.

## Patentansprüche

1. Dermales oder transdermales therapeutisches System mit einer Polymermatrix, wobei das Polymere wiederkehrende Einheiten mit einem Monomerbestandteil aus einem Zuckeralkohol oder einem Zuckeralkoholderivat als nachwachsendem Rohstoff umfasst und
(i) der Zuckeralkohol, als 1-Desoxy-1-amino-Zuckeralkohol; oder
(ii) das Zuckeralkoholderivat als 1-Desoxy-1-amino-Zuckeralkoholderivat
die Ester- oder Amidogruppen einer polymerisierten Acrylsäure bildet.

2. Dermales oder transdermales therapeutisches System nach Anspruch 1 mit Methacrylsäure als Acrylsäure.

3. Dermales oder transdermales therapeutisches System nach Anspruch 1 oder 2, wobei es sich bei dem zuckeralkoholderivat um ein Disaccharid handelt.

4. Dermales oder transdermales therapeutisches System nach Anspruch 3 mit Lactitol als Disaccharid.

5. Dermales oder transdermales therapeutisches System nach mindestens einem der Ansprüche 1 bis 4, bei dem die Hydroxygruppen des Zuckeralkohols oder des Zuckeralkoholderivats, soweit sie nicht mit der polymerisierten Acrylsäure verknüpft sind, mit Schutzgruppen versehen sind.

6. Dermales oder transdermales therapeutisches System nach Anspruch 5, bei dem die Schutzgruppen durch Acylgruppen gebildet werden, insbesondere durch Acetylgruppen.

7. Dermales oder transdermales therapeutisches System nach mindestens einem der Ansprüche 1 bis 6, bei dem die Acrylsäure mit einem oder mit mehreren Comonomeren polymerisiert ist.

8. Dermales oder transdermales therapeutisches System nach Anspruch 7 mit einem Acrylat als Comonomerem.

9. Dermales oder transdermales therapeutisches System nach Anspruch 8 mit Methylacrylat, Butylacrylat, Hexylacrylat oder 2-Ethylhexylacrylat als Comonomerem.

10. Dermales oder transdermales therapeutisches System nach mindestens einem der Ansprüche 1 bis 9 mit einer Matrix aus:
Poly-[(1desoxy-1-methacrylamido-glucitol)-co-(ethylacrylat)],
Poly-[(1-desoxy-1-methacrylamido-glucitol)-co-(butylacrylat)],
Poly-[(1-desoxy-1-methacrylamido-glucitol)-co-(hexylacrylat)],
Poly-[(1-desoxy-1-methacrylamido-glucitol)-co-(2-ethylhexylacrylat)],
Poly-[(penta-O-acetato-1-desoxy-1-methacrylamide-glucitol)-co-(ethylylacrylat)],
Poly-[(penta-O-acetato-1-desoxy-1-methacrylamido-glucitol)-co-(butylacrylat)],
Poly-[(penta-O-acetato-1-desoxy-1-methacrylamido-glucitol)-co-(hexylacrylat)],
Poly-[(penta-O-acelate-1-desoxy-1-methacrylamido-glucitol)-co-(2-ethylhcxylacrylat)].
Poly-[(1-desoxy-1-methacrylamido-lactitol)-co-(ethylacrylat)],
Poly-[(1-desoxy-1-methacrylamido-lactitol)-co-(butylacrylat)],
Poly-[(1-desoxy-1-methacrylamido-lactitol)-co-(hexylacrylat)],
Poly-[(1-desoxy-1-methacrylamido-lactitol)-co-(2-ethylhexylacrylat)],
Poly-[(penta-O-acetato-1-desoxy-1-methacrylamido-lactitol)-co-(ethylylacrylat)],
Poly-[(penta-O-acetato-1-desoxy-1-methacrylamido-lactitol)-co-(butylacrylat)],
Poly-[(penta-O-acetato-1-desoxy-1-methacrylamido-lactitol)-co-(hexylacrylat)] und/oder
Poly-[(penta-O-acetato-1-desoxy-1-methacrylamido-lactitol)-co-(2-ethylhexylacrylat)].

11. Dermales oder transdermales therapeutisches System nach mindestens einem der Ansprüche 1 bis 10, wobei die Matrix zusätzlich einen oder mehrere Weichmacher umfasst.

12. Dermales oder transdermales therapeutisches System nach Anspruch 11, wobei das System einen oder mehrere Weichmacher aus der durch Triethylenglykol (TEG), Triethylcitrat (TEC), Propylenglycol (PG), Glycerol (C), Triacetin (TriAc), Bis- (2-ethylhexyl)-adipat (EtHex), Diisobutyladipat (i-But) und Isopropylmyristat (IPM) gebildeten Gruppe umfasst.

13. Dermales oder transdermales therapeutisches System nach mindestens einem der Ansprüche 1 bis 12, wobei das System neben der Matrix eine Abdeckfolie und gegebenenfalls eine Abziehfolie umfasst.

14. Dermales oder transdermales therapeutisches System nach Anspruch 13, wobei die Abdeckfolie und/oder die Abziehfolie Folien auf Polyesterbasis sind.

15. Dermales oder transdermales therapeutisches System nach Anspruch 14, wobei die Abdeckfolie und/oder die Abziehfolie Folien auf Basis von Polyethylentherephthalat sind.

16. Dermales oder transdermales therapeutisches System nach mindestens einem der Ansprüche 1 bis 15, wobei die Matrix einen oder mehrere Wirkstoffe umfasst.

17. Dermales oder transdermales therapeutisches System nach Anspruch 16, wobei die Matrix Ibuprofen als Wirkstoff umfasst.

18. Dermales oder transdermales therapeutisches System nach, mindestens einem der Ansprüche 1 bis 17, wobei die Matrix neben den fakultativen Weichmachern einen oder mehrere übliche Hilfsstoffe umfasst.

## Claims

1. Dermal or transdermal therapeutic system having a polymer matrix, wherein the polymer comprises repeating units with a monomer component from a sugar alcohol or a sugar alcohol derivative as renewable raw material and
(i) the sugar alcohol as 1-desoxy-1-amino-sugar alcohol; or
(ii) the sugar alcohol derivative as 1-desoxy-1-amino-sugar alcohol derivative
forms the ester or amido groups of a polymerized acrylic acid.

2. Dermal or transdermal therapeutic system according to claim 1 with methacrylic acid as acrylic acid.

3. Dermal or transdermal therapeutic system according to claim 1 or 2, wherein the sugar alcohol derivative is a disaccharide.

4. Dermal or transdermal therapeutic system according to claim 3 with lactitol as disaccharide.

5. Dermal or transdermal therapeutic system according to at least one of claims 1 to 4, wherein the hydroxy groups of the sugar alcohol or the sugar alcohol derivative, to the extent to which they are not attached to the polymerized acrylic acid, are provided with protecting groups.

6. Dermal or transdermal therapeutic system according to claim 5, wherein the protecting groups are formed by acyl groups, especially acetyl groups.

7. Dermal or transdermal therapeutic system according to at least one of claims 1 to 6, wherein the acrylic acid is polymerized with one or more co-monomer(s).

8. Dermal or transdermal therapeutic system according to claim 7 with an acrylate as co-monomer.

9. Dermal or transdermal therapeutic system according to claim 8 with ethyl acrylate, butyl acrylate, hexyl acrylate or 2-ethylhexyl acrylate as co-monomer.

10. Dermal or transdermal therapeutic system according to at least one of claims 1 to 9 with a matrix selected from:
Poly-[(1-deoxy-1-methacrylamido-glucitol)-co-(ethyl acrylate)],
Poly-[(1-deoxy-1-methacrylamido-glucitol)-co-(butyl acrylate)],
Poly-[(1-deoxy-1-methacrylamido-glucitol)-co-(hexyl acrylate)],
Poly-[(1-deoxy-l-methacrylamido-glucitol)-co-(2-ethylhexyl acrylate)],
Poly-[(penta-O-acetato-1-deoxy-1-methacrylamido-glucitol)-co-(ethyl acrylate)],
Poly-[(penta-O-acetato-1-deoxy-1-methacrylamido-glucitol)-co-(butyl acrylate)],
Poly-[(penta-O-acetato-1-deoxy-1-methacrylamido-glucitol)-co-(hexyl acrylate)],
Poly-[(penta-O-acetato-1-deoxy-1-methacrylamido-glucitol)-co-(2-ethylhexyl acrylate)],
Poly-[(1-deoxy-1-methacrylamido-lactitol)-co-(ethyl acrylate)],
Poly-[(1-deoxy-1-methacrylamido-lactitol)-co-(butyl acrylate)],
Poly-[(1-deoxy-1-methacrylamido-lactitol)-co-(hexyl acrylate)],
Poly-[(1-deoxy-l-methacrylamido-lactitol)-co-(2-ethylhexyl acrylate)],
Poly-[(penta-O-acetato-1-deoxy-1-methacrylamido-lactitol)-co-(ethyl acrylate)],
Poly-[(penta-O-acetato-1-deoxy-1-methacrylamido-lactitol)-co-(butyl acrylate)],
Poly-[(penta-O-acetato-1-deoxy-1-methacrylamido-lactitol)-co-(hexyl acrylate)] and/or
Poly-[(penta-O-acetato-1-deoxy-1-methacrylamido-lactitol)-co-(2-ethylhexyl acrylate)].

11. Dermal or transdermal therapeutic system according to at least one of claims 1 to 10, wherein the matrix further comprises one or more softener(s).

12. Dermal or transdermal therapeutic system according to claim 11, wherein the system comprises one or more softener(s) from the group composed of triethylene glycol (TEG), triethyl citrate (TEC), propylene glycol (PG), glycerole (G), triacetin (TriAc), bis-(2-ethylhexyl)-adipate (EtHex), diisobutyladipate (i-But) and isopropylmyristate (IPM).

13. Dermal or transdermal therapeutic system according to at least one of claims 1 to 12, wherein the system comprises in addition to the matrix a backing layer and, optionally, a release liner.

14. Dermal or transdermal therapeutic system according to claim 13, wherein the backing layer and/or the release liner are sheets based on polyester.

15. Dermal or transdermal therapeutic system according to claim 14, wherein the backing layer and/or the release liner are sheets based on polyethylene therephthalate.

16. Dermal or transdermal therapeutic system according to at lease one of claims 1 to 15, wherein the matrix comprises one or more active ingredient(s).

17. Dermal or transdermal therapeutic system according to claim 16, wherein the matrix comprises ibuprofen as active ingredient.

18. Dermal or transdermal therapeutic system according to at least one of claims 1 to 17, wherein the matrix comprises in addition to the optional softeners one or more customary additives.

## Revendications

1. Système thérapeutique dermique ou transdermique comprenant une matrice de polymère, le polymère comprenant des motifs répétitifs présentant une teneur en monomères d'un alcool de sucre ou d'un dérivé d'alcool de sucre comme matière première renouvelable et
(i) l'alcool de sucre sous la forme d'alcool de sucre 1-désoxy-1-amino ; ou
(ii) le dérivé d'alcool de sucre sous la forme de dérivé d'alcool de sucre 1-désoxy-1-amino
forme les groupes esters ou amido d'un acide acrylique polymérisé.

2. Système thérapeutique dermique ou transdermique selon la revendication 1 comportant de l'acide méthacrylique en tant qu'acide acrylique.

3. Système thérapeutique dermique ou transdermique selon la revendication 1 ou 2, dans lequel le dérivé d'alcool de sucre est un disaccharide.

4. Système thérapeutique dermique ou transdermique selon la revendication 3, comportant du lactitol en tant que disaccharide.

5. Système thérapeutique dermique ou transdermique selon au moins l'une des revendications 1 à 4, dans lequel les groupes hydroxy de l'alcool de sucre ou du dérivé d'alcool de sucre, dans la mesure où ils ne sont pas reliés à l'acide acrylique polymérisé, sont dotés de groupes protecteurs.

6. Système thérapeutique dermique ou transdermique selon la revendication 5, dans lequel les groupes protecteurs sont formés par des groupes acyle, en particulier par des groupes acétyle.

7. Système thérapeutique dermique ou transdermique selon au moins l'une des revendications 1 à 6, dans lequel l'acide acrylique est polymérisé avec un ou plusieurs co-monomères.

8. Système thérapeutique dermique ou transdermique selon la revendication 7, comportant un acrylate comme co-monomère.

9. Système thérapeutique dermique ou transdermique selon la revendication 8, comportant de l'éthylacrylate, du butylacrylate, de l'hexylacrylate ou du 2-éthylhexylacrylate comme co-monomère.

10. Système thérapeutique dermique ou transdermique selon au moins l'une des revendications 1 à 9, comportant une matrice de
poly-[(1-désoxy-1-méthacrylamido-glucitol)-co-(éthylacrylate)],
poly-[(1-désoxy-1-méthacrylamido-glucitol)-co-(butylacrylate)],
poly-[(1-désoxy-1-méthacrylamido-glucitol)-co-(hexylacrylate)],
poly-[(1-désoxy-1-méthacrylamido-glucitol)-co-(2-éthylhexyl-acrylate)],
poly-[(penta-O-acétato-1-désoxy-1-méthacrylamidoglucitol)-co-(éthylacrylate)],
poly-[(penta-O-acétato-1-désoxy-1-méthacrylamidoglucitol)-co-(butylacrylate)],
poly-[(penta-O-acétato-1-désoxy-1-méthacrylamidoglucitol)-co-(hexylacrylate)],
poly-[(penta-O-acétato-1-désoxy-1-méthacrylamidoglucitol)-co-(2-éthylhexylacrylate)],
poly-[(1-désoxy-1-méthacrylamido-lactitol)-co-(éthylacrylate)],
poly-[(1-désoxy-1-méthacrylamido-lactitol)-co-(butylacrylate)],
poly-[(1-désoxy-1-méthacrylamido-lactitol)-co-(hexylacrylate)],
poly-[(1-désoxy-1-méthacrylamido-lactitol)-co-(2-éthylhexylacrylate)],
poly-[(penta-O-acétato-1-désoxy-1-méthacrylamidolactitol)-co-(éthylacrylate)],
poly-[(penta-O-acétato-1-désoxy-1-méthacrylamidolactitol)-co-(butylacrylate)],
poly-[(penta-O-acétato-1-désoxy-1-méthacrylamidolactitol)-co-(hexylacrylate)], et/ou
poly-[(penta-O-acétato-1-désoxy-1-méthacrylamidolactitol)-co-(2-éthylhexylacrylate)].

11. Système thérapeutique dermique ou transdermique selon au moins l'une des revendications 1 à 10, dans lequel la matrice comprend en outre un ou plusieurs plastifiants.

12. Système thérapeutique dermique ou transdermique selon la revendication 11, le système comprenant un ou plusieurs plastifiants choisis dans le groupe formé par le triéthylène glycol (TEG), le triéthylcitrate (TEC), le propylène glycol (PG), le glycérol (G), la triacétine (TriAc), le bis-(2-éthylhexyl)-adipate (Etilex), le diisobutyladipate (i-but) et l'isopropylmyristate (IPM).

13. Système thérapeutique dermique ou transdermique selon au moins l'une des revendications 1 à 12, le système comprenant, outre la matrice, une feuille de recouvrement et éventuellement, une feuille de décollement.

14. Système thérapeutique dermique ou transdermique selon la revendication 13, dans lequel la feuille de recouvrement et/ou la feuille de décollement sont à base de polyester.

15. Système thérapeutique dermique ou transdermique selon la revendication 14, dans lequel la feuille de recouvrement et/ou la feuille de décollement sont à base de polyéthylène-téréphtalate.

16. Système thérapeutique dermique ou transdermique selon au moins l'une des revendications 1 à 15, dans lequel la matrice comprend une ou plusieurs substances actives.

17. Système thérapeutique dermique ou transdermique selon la revendication 16, dans lequel la matrice comprend de l'ibuprofène comme substance active.

18. Système thérapeutique dermique ou transdermique selon au moins l'une des revendications 1 à 17, dans lequel la matrice comprend, outre le plastifiant facultatif, un ou plusieurs adjuvants classiques.
